(19) Europäisches Patentamt
European Patent Office
Office européen des brevets

(11) EP 4 050 093 A1

(12) **EUROPEAN PATENT APPLICATION**
published in accordance with Art. 153(4) EPC

(43) Date of publication:
31.08.2022 Bulletin 2022/35

(21) Application number: 20878084.1

(22) Date of filing: 15.10.2020

(51) International Patent Classification (IPC):
$C12M\ 3/06^{(2006.01)}$    $B01D\ 15/00^{(2006.01)}$
$B01J\ 19/00^{(2006.01)}$    $G01N\ 33/48^{(2006.01)}$

(52) Cooperative Patent Classification (CPC):
B01D 15/00; B01J 19/00; C12M 3/06; G01N 33/48

(86) International application number:
PCT/JP2020/038986

(87) International publication number:
WO 2021/079826 (29.04.2021 Gazette 2021/17)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.10.2019 JP 2019192420

(71) Applicant: TL Genomics Inc.
Koganei-shi, Tokyo 184-0012 (JP)

(72) Inventors:
• KUBO, Tomohiro
Koganei-shi, Tokyo 184-0012 (JP)
• YAMANAKA, Hiroaki
Koganei-shi, Tokyo 184-0012 (JP)

(74) Representative: Papula Oy
P.O. Box 981
00101 Helsinki (FI)

(54) **DEVICE FOR BLOOD**

(57) A device for blood (1) is provided with a column (50) and a micro flow path (20) located downstream of the column (50). The column (50) includes a porous material as a solid phase, and blood that has contacted with the porous material flows through the micro flow path (20). In the device for blood (1), the column (50) and the micro flow path (20) are provided as separated bodies. The column (50) has a connecting part (55), the micro flow path (20) has an inlet (21a), the connecting part (55) and the inlet (21a) are connected to each other to integrate the column (50) with the micro flow path (20), and blood (BL) is allowed to pass from the column (50).

FIG. 1

EP 4 050 093 A1

**Description**

Technical Field

The present invention relates to a device for blood, particularly to a device for blood cell separation (hereinafter also referred to as "blood cell separation device"). The present invention also relates to a method for using the devices.

Background Art

Patent Literature (hereinafter, referred to as PTL) 1 describes classification of blood cells by using a microchannel.

Citation List

Patent Literature

PTL 1 WO2018/123220

Summary of Invention

Technical Problem

Blood flowing through a microchannel may cause clogging in the microchannel. An object of the present invention is to provide a device suitable for eliminating or reducing such clogging and a method for using the device.

Solution to Problem

< 1 > A device for blood, the device including: a column; and a microchannel located downstream of the column, in which:

the column includes a porous material as a stationary phase, and
blood flows through the microchannel after contacted with the porous material.

< 2 > The device for blood according to < 1 >, in which:

the porous material is composed of particles;
the column further includes a housing part and a filter, in which the housing part is for housing the porous material, the filter is for trapping the particles of the porous material, and the filter is located downstream of the housing part on one side of the housing part, the one side being closer to the microchannel than the other side of the housing part is;
a small particle having a particle size equal to or smaller than a cutoff diameter is removed in advance from the particles of the porous material; and
an opening of the filter is smaller than the cutoff diameter.

< 3 > The device for blood according to < 2 >, in which the cutoff diameter is in a range of 25 $\mu$m to 100 $\mu$m.
< 4 > The device for blood according to < 2 > or < 3 >, in which a diameter of a mesh of the filter is in a range of 20 $\mu$m to 40 $\mu$m, and the range is less than the cutoff diameter.
<5 > The device for blood according to any one of < 2 > to < 4 >, in which:

the particles of the porous material have a particle size distribution; and
median particle size (d50V) of the particles of the porous material in a volume-based cumulative distribution is 25 to 280 $\mu$m, in which the particle size distribution represents a particle size distribution before the small particle is removed according to the cutoff diameter.

< 6 > The device for blood according to any one of < 2 > to < 5 >, in which:
the column further includes a filter for trapping the particles of the porous material, the filter being located upstream of the housing part on the other side of the housing part, the other side being farther from the microchannel than the one side of the housing part is.

< 7 > The device for blood according to any one of < 2 > to < 6 >, in which:

when the blood flows through the microchannel after contacted with the porous material, the microchannel hydraulically classifies blood cells in the blood.

< 8 > The device for blood according to < 7 >, in which:

the device for blood is for blood cell separation;
the microchannel is made of a flat chip, the flat chip including a pillar dense area and a hydraulic channel located downstream of the pillar dense area;
the column is connected to a front or a back of the flat chip; and
the device for blood further includes an outlet for discharging the hydraulically classified blood cells from the microchannel to an outside of the device for blood.

< 9 > The device for blood according to any one of < 1 > to < 8 >, in which:

the porous material is composed of particles; and
the particles of the porous material have a porous surface made of a polysaccharide, silica, or a resin.

< 10 > A method for using the device for blood according to any one of < 1 > to < 9 >, the method including: allowing blood to enter the microchannel from the column, in which

the column and the microchannel are provided as separate bodies in the device for blood,
the column includes a coupling part,
the microchannel includes an inlet, and
the blood is allowed to enter the microchannel after the coupling part is coupled with the inlet to integrate the column with the microchannel.

Advantageous Effects of Invention

The present invention is capable of providing a device suitable for eliminating or reducing clogging in a microchannel and a method for using the device.

Brief Description of Drawing

FIG. 1 is a schematic view of a device for blood separation;
FIG. 2 is a front view of a column;
FIG. 3 illustrates the particle size distribution of porous particles;
FIG. 4 is a front cross-sectional view of the device for blood separation;
FIG. 5 is an enlarged front cross-sectional view of the column shown in area V of FIG. 4;
FIG. 6 illustrates observation image 1 (upper row) of the microchannel, namely an Example, shown in area VI of FIG. 1 and the sketch thereof (lower row);
FIG. 7 is a partial plan view of the microchannel shown in area VII of FIG. 1;
FIG. 8 illustrates observation image 2 (upper row) of a microchannel according to Comparative Example 1 and the sketch thereof (lower row);
FIG. 9 illustrates observation image 3 (upper row) of a microchannel according to Comparative Example 2 and the sketch thereof (lower row); and
FIG. 10 is a cross-sectional view of a microchannel of a Reference Example.

Description of Embodiments

1. Device for Blood Separation

One aspect of the present invention relates to a device for blood separation (herein also referred to as "blood separation device") including a column and a microchannel located downstream of the column. Blood contains cells and blood plasma. The cells include blood cells and other cells that circulate in the blood. The cells in blood are a mixture of cells of various sizes. Each cell type exhibits a unique particle size distribution with respect to cell size. The blood separation device is for classifying cells in blood according to the size of the cells. Classifying blood by using the blood separation device can obtain a cell population having enriched cells of a specific cell type. An example of the classification is hydraulic classification performed in a microchannel.

Examples of the types of target blood to be classified by using the blood separation device and types of cells to be enriched are as follows.

Target blood contains blood cells as the cell type to be enriched. The blood cells may be nucleated red blood cells from a fetus (hereinafter referred to as "fetal nucleated red blood cells"). The blood to be obtained contains fetal nucleated red blood cells. Fetal nucleated red blood cells are contained in maternal blood. Fetuses and pregnant women are subjects to be diagnosed. A column is used to pretreat the maternal blood. The fetal nucleated red blood cells are enriched by classification with the use of a microchannel. Data useful for diagnosis of the fetus is obtained from the enriched fetal nucleated red blood cells.

Target blood contains other cells that circulate in the blood and are not blood cells as the cell type to be enriched. Such cells may be circulating tumor cells (CTCs). The blood to be obtained contains CTCs. The blood of subjects suspected of having cancer, cancer patients, and subjects who have already been treated for cancer, for example, may contain CTCs. These subjects and patients are subjects to be diagnosed. A column is used to pretreat the obtained blood. The CTCs are enriched by classification with the use of a microchannel. Steps necessary for enriching CTCs are carried out regardless of whether or not the blood contains the CTCs. Data useful for diagnosis of cancer is obtained from the enriched CTCs.

The cell type to be enriched that are contained in the target blood may be myeloma cells. The blood to be obtained contains myeloma cells. Myeloma cells may be detected as minimal residual disease (MRD) from, for example, patients treated for myeloma. These patients are subjects to be diagnosed. An example of myeloma is multiple myeloma. A column is used to pretreat blood collected from such a patient. The myeloma cells are enriched by classification. Steps necessary for enriching myeloma cells are carried out regardless of whether or not the blood contains the myeloma cells. Data useful for diagnosis of MRD is obtained from the enriched myeloma cells.

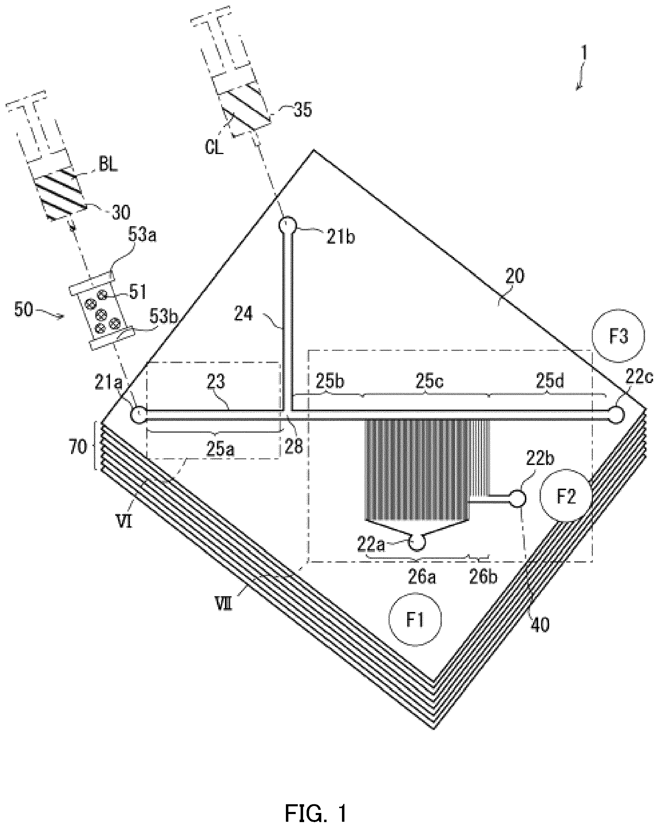

The following describes an example of a device to be used for hydraulic classification. Blood separation device 1 will be described as a whole with reference to the schematic view of FIG. 1. FIG. 1 illustrates blood separation device 1.

As illustrated in FIG. 1, blood separation device 1 includes column 50 and microchannel 20. As illustrated in FIG. 1, channel chip 70 having a multi-layer structure may be provided by stacking plurality of microchannels 20. Channel chip 70 may be composed of only one layer of microchannel 20. Hereinafter, microchannel 20 of the uppermost layer of channel chip 70 will be described. A layer below the uppermost layer of channel chip 70 may include a microchannel substantially the same as microchannel 20 described below, or may include a different microchannel. Column 50 and microchannel 20 may be configured as separate bodies. Column 50 is disposed upstream of microchannel 20.

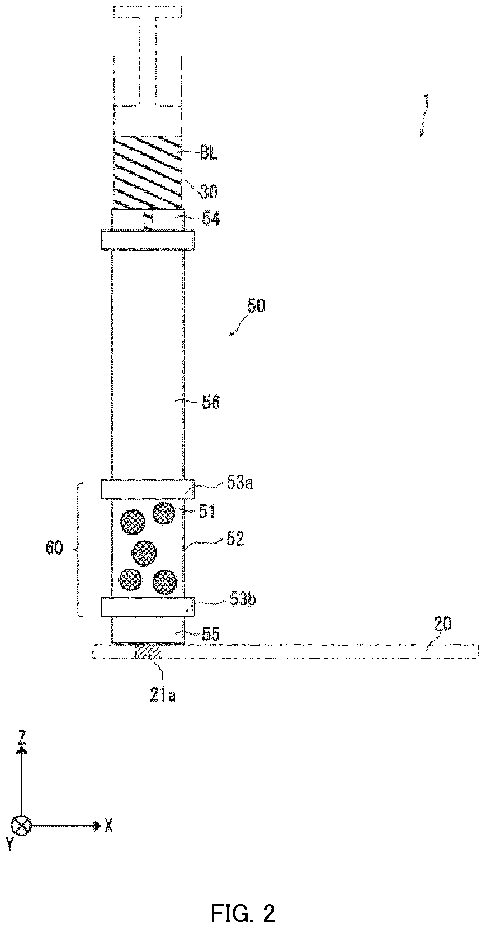

As illustrated in FIG. 1, one end of column 50 is coupled with syringe 30 containing blood BL. The other end of column 50 is coupled with inlet 21a of microchannel 20. Column 50 includes at least porous material 51 and filters 53a and 53b. In one aspect, column 50 is for performing column chromatography using porous material 51 filling the column as a stationary phase and blood BL as a mobile phase. Column 50 will be described in detail with reference to FIG. 2. Blood BL is brought into contact with the porous surface of porous material 51, thereby allowing porous material to react and interact with the components in blood BL to pretreat blood BL. Sending pretreated blood BL from syringe 30 at a predetermined flow rate allows the blood to pass through column 50. Thus-obtained pretreated blood BL enters inlet 21a of microchannel 20.

Microchannel 20 is used for separating floating cells such as blood cells. Microchannel 20 illustrated in FIG. 1 has a channel structure on the order of micrometers. Such a microchannel structure is suitable for blood classification (PTL 1). A chip including a microchannel to be used for blood classification may be particularly referred to as a blood cell separation chip or a channel chip. Blood BL is classified in microchannel 20 and reaches outlets 22a to 22c.

In FIG. 1, microchannel 20 includes main channel 23. One end of main channel 23 serves as inlet 21a. The other end of main channel 23 serves as outlet 22c. Microchannel 20 further includes sub channel 24. One end of sub channel 24 serves as inlet 21b. The other end of sub channel 24 is connected to main channel 23 at junction 28.

In FIG. 1, main channel 23 includes channel parts 25a to 25d provided sequentially from inlet 21a toward outlet 22c. Channel parts 25a to 25d are connected to each other to form one body from inlet 21a to outlet 22c. Junction 28 is located between channel part 25a and channel part 25b.

In FIG. 1, microchannel 20 includes branch channels 26a and 26b. Each of branch channels 26a and 26b branches off from main channel 23. Branch channels 26a and 26b branch off from main channel 23 in this order from the upstream side. One end of each of branch channels 26a and 26b is connected to main channel 23 at channel part 25c. In channel part 25c, branch channels 26a and 26b are disposed on the side opposite to sub channel 24. Outlet 22a and outlet 22b are located at the other ends of branch channels 26a and 26b.

In FIG. 1, each of branch channels 26a and 26b includes a plurality of small channels branching off from main channel 23. Small channels are disposed along the direction from the upstream to the downstream in main channel 23.

Each small channel reaches outlet 22a or 22b. Small channels join together immediately before outlets 22a or 22b. Channel part 25d is located downstream of channel part 25c. Channel part 25d reaches outlet 22c.

Blood BL is sent from syringe 30 to column 50 at a predetermined flow rate. Blood BL sent to column 50 enters channel part 25a via inlet 21a.

In FIG. 1, microchannel 20 includes sub channel 24. Sub channel 24 is coupled with syringe 35. Clarified liquid CL is housed in syringe 35. Clarified liquid CL is free from floating cells. Clarified liquid CL does not damage blood cells and other cells. Clarified liquid CL is a buffer. Clarified liquid CL may be PBS. Increasing pressure inside syringe 35 allow clarified liquid CL to enter sub channel 24 through inlet 21b. Clarified liquid CL flows through sub channel 24, and then flows into channel part 25b.

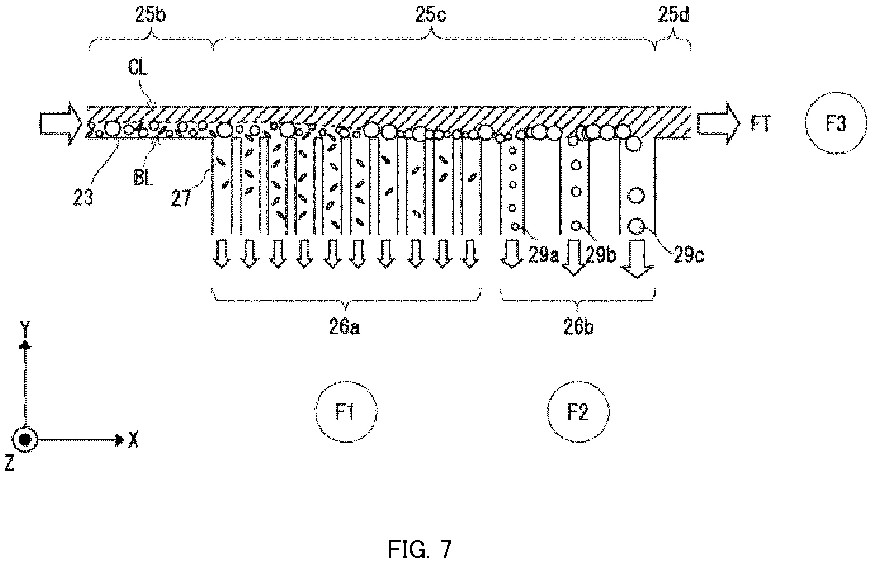

A fraction of a cell suspension is discharged through each outlet. Fraction F3, fraction F2, and fraction F1 are respectively obtained at outlet 22c, outlet 22b, and outlet 22a. Fraction F1 and fraction F2 each contain cells classified in channel part 25c. Fraction F3 contains blood plasma that has passed through channel part 25c. The details of the classification process of the floating cells that have passed through channel parts 25b to 25d shown in the area VII of FIG. 1 will be described below with reference to FIG. 7.

2. Details of Column

In the following, the details of column 50 will be described with reference to FIG. 2. Hereinafter, axes X, Y, and Z in the drawings are shown for convenience in order to understand the configuration and functions of column 50.

FIG. 2 illustrates column 50 viewed from the front. Column 50 includes column body 60. Column body 60 includes at least porous material 51, housing part 52 for housing porous material 51, and at least one filter (53a, 53b). Column 50 may further include coupling parts 54 and 55. Coupling part 54 can be attached to or detached from syringe 30. Coupling part 55 can be attached to or detached from inlet 21a of microchannel 20. Tube 56 may be provided between filter 53a and coupling part 54.

In the following, the details of each configuration will be explained.

2-1. Porous Material

Porous material 51 is a material whose surface is porous. In other words, a large number of micropores are formed in the surface of porous material 51. Porous material 51 may be particles. The particles may be spherical. The particles may be beads. As used herein, beads refer to a group of particles formed by a technique such that each particle is formed to have a spherical shape. Porous material 51 may sink in blood. It is preferable that small particles are cut off from the particles to be used as porous material 51 as needed. The cutting off (cutoff) herein means to remove small particles having a particle size (i.e., particle diameter) equal to or smaller than the cutoff diameter from the particles of the porous material in advance. Specifically, the removal can be performed by sieving with a mesh or the like. Details on the cutoff of the porous material will be described in "3. Cutoff of Particles of Porous Material."

The blood to be brought into contact with the porous surface of porous material 51 may be whole blood that is not diluted with another liquid. Whole blood means that the blood is not separated for each blood component and contains all components such as blood cells and blood plasma. For example, porous material 51 may interact with some of the floating cells contained in whole blood. The interacting components themselves may cause clogging of microchannel 20. The interacting components may indirectly promote clogging of microchannel 20.

The porous material may react with the components contained in blood plasma. For example, the porous material may interact with the components contained in blood plasma.

The porous material may be bonded to another material that is non-porous. For example, non-porous particles may be coated with a porous material to form porous particles. The center of each particle may be non-porous. The center of each particle may be ferromagnetic.

The material of the porous material may be polysaccharides. The part with micropores in the porous material may be formed of polysaccharides. The polysaccharide may be crosslinked. The polysaccharides may be any of agarose, dextran, and allyl dextran. The polysaccharides may be modified. The modification may be DEAE (Diethylethanolamine) modification. In addition, the porous material may be made of silica or resin.

The particulate porous material may be a material that can be used for gel filtration chromatography. Gel filtration chromatography is size exclusion chromatography using an aqueous solution as the mobile phase thereof. A material that can fractionate DNA may be employed for the chromatography. The exclusion limit of the porous material for DNA is preferably 45 base pairs or more. The exclusion limit of the porous material for DNA may be 165 base pairs or more or 165 base pairs or less. The exclusion limit of the porous material for DNA may be 1078 base pairs or more or 1078 base pairs or less.

The particulate porous material may be a material that can fractionate a protein. The lower limit of the fractionation range of the porous material with respect to the protein is preferably $1 \times 10^4$ Da or more. The upper limit of the fractionation range of the porous material with respect to the protein is preferably $4 \times 10^6$ Da or more. The particulate porous material preferably satisfies at least any one of the aforementioned conditions.

2-2. Housing Part

As illustrated in FIG. 2, housing part 52 is configured to be capable of housing porous material 51. Housing part 52 may have a shape such that end surfaces thereof are open at both ends of the housing part, and can be, for example, a hollow form having a shape of a cylinder or a square cylinder in a cross-sectional view. For example, housing part 52 having a cylindrical shape includes no corner part; therefore, the retention of porous material 51 and the flow of blood BL at the corner part can be prevented. It is thus particularly preferable that the shape of housing part 52 is cylindrical. When the shape of housing part 52 is cylindrical, the cross-sectional shape of housing part 52 may be any of, for example, a perfect circle, a substantially perfect circle, and an ellipse.

2-3. Filter

Filters 53a and 53b have a mesh structure such that particles of the porous material cannot pass therethrough, but a desired liquid such as blood to be classified in a microchannel can pass therethrough. That is, filters 53a and 53b trap the particles of the porous material. Small particles having a particle size equal to or smaller than the cutoff diameter are removed from the particles of the porous material in advance, and thus small particles having a certain particle size or less are excluded. Therefore, filters 53a and 53b preferably have a mesh structure such that opening of the filter is smaller than the cutoff diameter of the particles of the porous material, that is, smaller than the minimum particle size in the particles of the porous material, and cells such as blood cells can pass through the filter. The size of a cell is, for example, about 12 $\mu$m at the maximum. Thus, a filter with a mesh structure having, for example, a diameter of 20 to 40 $\mu$m, particularly preferably a diameter of 20 $\mu$m, can be used. It is preferable that the diameter of the mesh structure is constant. Providing such a mesh structure for filters 53a and 53b can prevent the particles of the porous material from leaking to the outside of column body 60.

Herein, the "diameter of mesh structure" means the opening of filters 53a and 53b. Examples of the mesh structure include structures in which straight lines are disposed so as to intersect each other in a crisscross pattern, and structures in which polygons are repeatedly disposed. Examples of the structure in which straight lines are disposed so as to intersect each other in a crisscross pattern include grid-like structures. The mesh structure may include repeatedly disposed circles.

Of the both ends of housing part 52, the filter (as shown as filter 53b) can be disposed at least one end on the side where inlet 21a of microchannel 20 is located. That is, filter 53b can be disposed on the downstream side in the blood flow in column 50. In addition, filters 53a and 53b can also be disposed at the both ends of housing part 52. That is, filters 53a and 53b can be disposed on the upstream side and the downstream side in the blood flow in column 50. Filters 53a and 53b can be disposed so as to completely cover the entire surface of the open end surfaces located at both ends of housing part 52. It is preferable that filters 53a and 53b each have an outer diameter at least equal to or larger than the outer diameter of housing part 52 in order to completely cover the entire surfaces of the open end surfaces of housing part 52. By disposing filters 53a and 53b on both end surfaces of housing part 52, leaking of porous material 51 to the outside can be prevented while blood BL is brought into contact with porous material 51 filling housing part 52.

2-4. Coupling Part

Coupling parts 54 and 55 are members for coupling column body 60 with a syringe located upstream of column 50 and with a microchannel located downstream of column 50. As illustrated in FIG. 2, coupling part 54 is configured in such a way that the coupling part can be attached to or detached from syringe 30. Coupling part 55 is configured in such a way that the coupling part can be attached to or detached from inlet 21a of microchannel 20. Coupling parts 54 and 55 may have any shape, but it is preferable that coupling parts 54 and 55 are configured to be easily attached to or detached from the corresponding components. For example, coupling part 54 may have a shape such that the coupling part includes a concave portion that fits to a convex-shaped portion at the tip of syringe 30.

For example, coupling part 55 may have a shape such that coupling part 55 includes a convex portion corresponding to a concave portion of inlet 21a of microchannel 20. Further, it is also possible to provide screw-shaped grooves on the surfaces of the convex portion and the concave portion so that the portions can be rotated relative to each other and connected to each other. Such a configuration can securely couple column 50 with microchannel 20, thereby improving the operability.

The shapes of coupling parts 54 and 55 may be configured, for example, in such a way that the coupling part cannot be detached once the coupling part is coupled with syringe 30 or inlet 21a. With this undetachable configuration, the coupling can be made more securely, and thus when the pressure from syringe 30 is applied to each coupling part, the blood flowing through the coupling part does not easily leak to the outside. The watertightness at the coupling part thus can be improved.

2-5. Tube

In one aspect, tube 56 is provided between filter 53a and coupling part 54 as illustrated in FIG. 2. Tube 56 may have flexibility, and for example, a silicone resin tube, a polyvinyl chloride tube, or the like can be used as tube 56. Providing tube 56 can give some distance from the syringe that feeds blood to column body 60. For example, tube 56 may be provided with a pinch valve. For disposing column 50 at a position in microchannel 20, another tube may be provided between filter 53b and coupling part 55. When the distance from syringe 30 to column body 60 is short, provision of tube 56 may not be required. When tube 56 is not provided but coupling part 54 described in "2-4. Coupling Part" is provided, a configuration such that coupling part 54 is directly coupled with filter 53a is also possible.

3. Cutoff of Particles of Porous Material

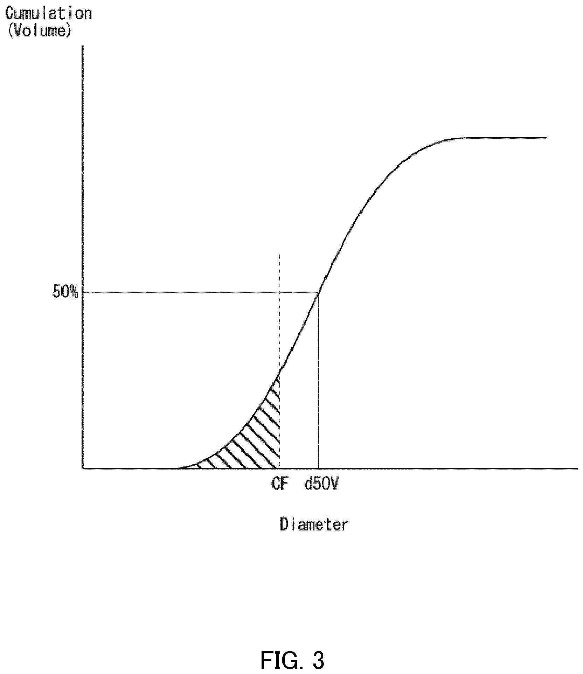

In the following, the particle size distribution of porous particles will be described with reference to FIG. 3, and then the cutoff of the particles of a porous material will be described in more detail. The cutoff means to remove small particles from the particles of the porous material.

FIG. 3 illustrates the particle size distribution of porous particles as a volume-based cumulative distribution. The particles of a porous material have a particle size distribution. The median particle size d50V (median particle size of the cumulative volume distribution) of the porous material is the median particle size in the volume-based cumulative distribution. When the particles are made of a polysaccharide, the particle size of the particles that have swelled in a buffer is used as a reference. A measurement example of the particle size is an effective size determined by laser diffraction or light-scattering, or a sphere volume equivalent diameter determined by the Coulter method.

In FIG. 3, median particle size d50V is preferably less than 500 $\mu$m. The median particle size d50V is preferably 25 to 280 $\mu$m, more preferably 25 to 165 $\mu$m, and further preferably 45 to 165 $\mu$m. The median particle size d50V falling within such a range enables the surface area of the porous material to be suitable for the contact with blood.

In FIG. 3, small particles are preferably cut off from the particles of the porous material, as needed. In one aspect, small particles having a particle size equal to or smaller than the cutoff diameter CF are removed in advance. That is, the cutoff diameter CF means the particle size of the minimum particle in the particles of the porous material. The range of the cutoff diameter CF is 25 to 100 $\mu$m. The range of the cutoff diameter CF may be 25 to 70 $\mu$m, preferably 40 to 70 $\mu$m. The removal of small particles is preferably performed by sieving with a mesh. For example, a cell strainer may be used for removing small particles from a population of the particles of the porous material. With small particles being removed, clogging in a microchannel caused by small particles of the porous material mixed in the blood can be prevented.

Depending on the type of microchannel, small particles of a porous material that have entered the microchannel can flow out of the microchannel without causing any problem. For such a microchannel, clogging caused by small particles may not necessarily be taken into consideration. However, even in such a microchannel, clogging (debris described below) may still occur due to some chemical components in the blood. Even for such a microchannel, employing particles of a porous material is also effective for preventing clogging, regardless of whether or not small particles are cut off in the particles of the porous material.

When small particles are removed from the original particles, the particle size distribution of the particles changes from the original. That is, the cutoff has an effect of size selection. After the size selection, the median particle size also changes. For convenience, the median particle size d50V herein is based on the particle size distribution before small particles are removed from the original particles by the cutoff.

The device for blood separation according to the present invention has been described.

The column and the microchannel may be configured as separate bodies. At the time of use, the column and the microchannel can be combined and used as one blood cell separation device. The column and the microchannel can be coupled with each other by using a coupling part having a concave or convex structure as described above, for example. In addition, the column and the microchannel can be coupled with each other by using a coupling part including screw-shaped grooves on the surface of the concave or convex structure.

Alternatively, the column and the microchannel may be configured as one body. For example, a structure including a column structure coupled with the inlet of the microchannel is possible. The blood separation device according to the present invention may be a cartridge integrated with a syringe. Further, for the use, this cartridge may be connected to a driving device for driving the microchannel, or disposed inside the driving device.

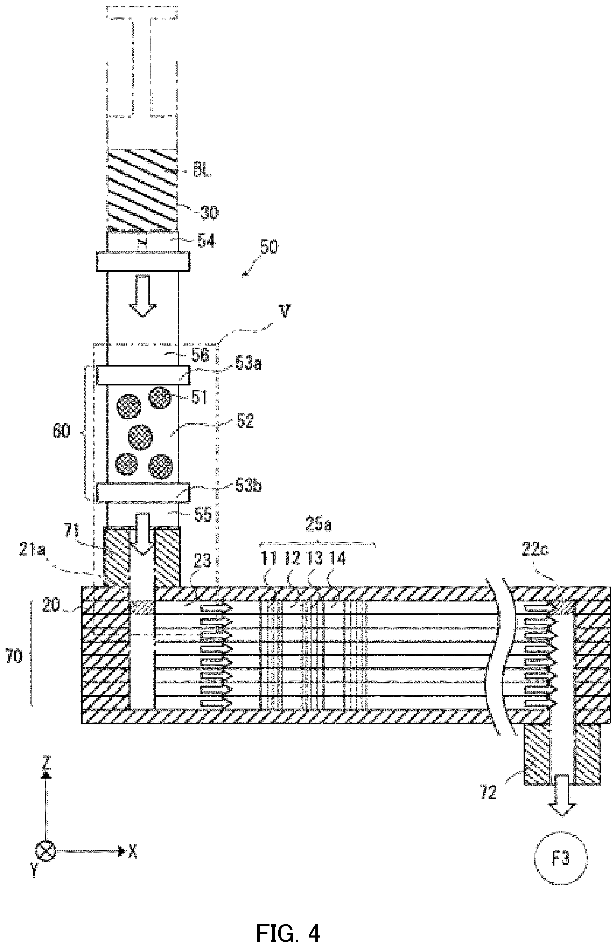

Hereinafter, the details of the blood classification process using the blood separation device will be described with reference to FIGS. 4 to 7.

4. Outline of Blood Flow

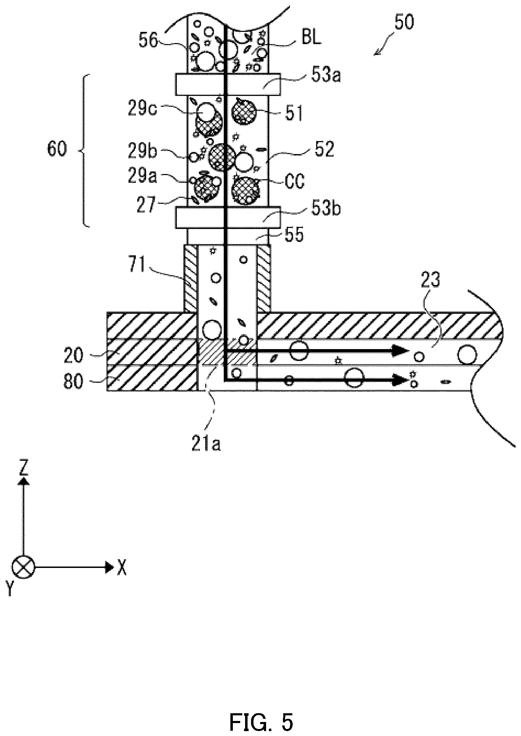

In the following, the blood flow inside the blood separation device will be described with reference to FIG. 4. In the drawing, the blood separation device is in a cross-sectional view from the front side. White arrows indicate a series of blood BL flows. Channel chip 70 of this example includes eight layers composed of microchannel 20 and microchannels substantially the same as microchannel 20. The uppermost layer of channel chip 70 is composed of a plate-shaped sheet to which inlet 71 to be connected to inlet 21a of microchannel 20 is attached. Inlet 21a of microchannel 20 and the inlets of the other microchannels in channel chip 70 are combined into inlet 71. Channel chip 70 includes, in the lowermost layer thereof, outlet 72 to be connected to outlet 22c of microchannel 20. Outlet 22c of microchannel 20 and the outlets of the other microchannels in channel chip 70 are combined into outlet 72. As indicated by the white arrows, blood BL sent from syringe 30 passes through column 50. The behavior of blood BL in column 50 will be described below with reference to FIG. 5.

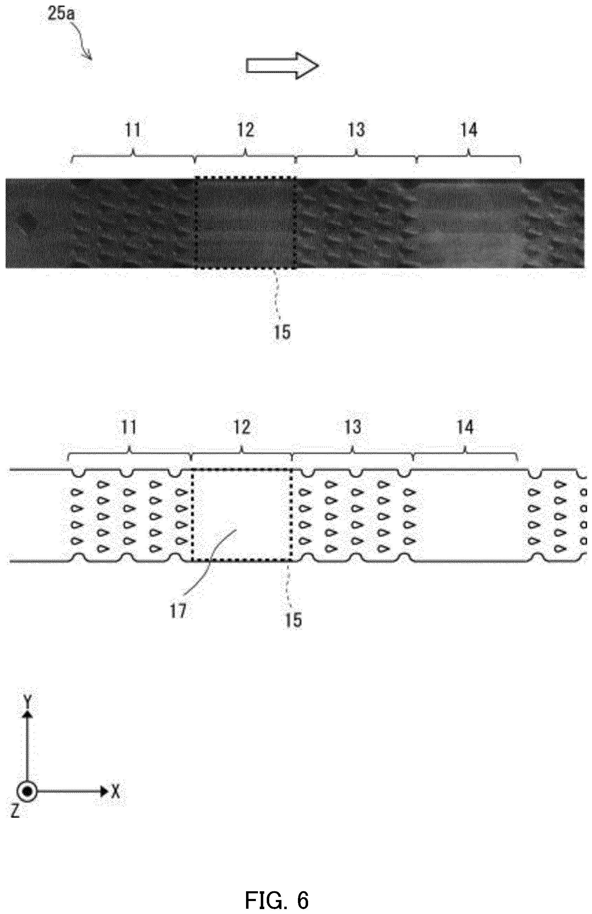

In the following, microchannel 20 of the uppermost layer in channel chip 70 will be described. The other layers also have a configuration substantially the same as microchannel 20. As illustrated in FIG. 4, blood BL that has passed through column 50 and inlet 71 enters microchannel 20 from inlet 21a. Blood BL that has entered microchannel 20 enters main channel 23, which is formed in each layer of stacked microchannels 20. Channel part 25a includes pillar dense areas 11 and 13 acting as filters, and sections 12 and 14. In FIG. 4, pillar dense areas 11 and 13 are indicated by vertical hatching. Channel part 25a will be described in detail with reference to FIG. 6. That is, microchannel 20 is made of a flat chip including pillar dense areas 11 and 13 and a hydraulic channel located downstream of pillar dense areas 11 and 13.

In FIG. 4, the blood that has passed through channel part 25a subsequently passes through channel parts 25b to 25d illustrated in FIG. 1. As a result, microchannel 20 classifies the floating cells contained in the blood. The microchannels of the lower layers in channel chip 70 also classify the floating cells in substantially the same manner. Among the classified floating cells, fraction F3 reaches outlet 72 via outlet 22c. Details of the process of classifying the floating cells will be described below with reference to FIG. 7.

5. Interaction between Column and Blood

In the following, the interaction between a column and blood will be described with reference to FIG. 5. FIG. 5 is an enlarged front cross-sectional view of column 50 shown in area V of FIG. 4. FIG. 5 illustrates the interaction between the column and blood.

In FIG. 5, for simplifying the explanation, only a total of two layers, that is, microchannel 20 and microchannel 80 that represents the channels below microchannel 20, are shown.

In FIG. 5, the blood flow is indicated by black arrows. Blood BL contains non-nucleated red blood cells 27, nucleated cells 29a to 29c, and precipitation-causing substance CC. Nucleated cells 29a to 29c are cells of different sizes. Precipitation-causing substance CC is considered to be one of the causes of clogging (debris) of microchannel 20 and microchannel 80.

As illustrated in FIG. 5, blood BL sent from syringe 30 at a predetermined flow rate passes through column body 60 of column 50. As illustrated in FIG. 5, precipitation-causing substance CC passes through porous material 51. Porous material 51 interacts with precipitation-causing substance CC contained in the blood to significantly reduce the precipitation property of the precipitation-causing substance. It is preferable to use porous material 51 at an amount sufficient for reducing the precipitation property of precipitation-causing substance CC so that that precipitation-causing substance CC forms substantially no gel-like debris in microchannel 20. The prevention of clogging (debris) in microchannel 20 and other microchannels may be made by porous material 51 adsorbing precipitation-causing substance CC. On the other hand, non-nucleated red blood cells 27 and nucleated cells 29a to 29c flow through the gaps in porous material 51. Porous material 51 thus does not block the flow of blood cells.

6. Pillar Dense Area

The blood that has passed through column 50 subsequently passes through pillar dense area 11 provided in main channel 23. In the following, a pillar dense area will be described with reference to FIG. 6.

FIG. 6 illustrates the observation image (upper row) of the microchannel shown in area VI of FIG. 1 and the sketch thereof (lower row). Pillar dense area 11 will be described with reference to FIG. 6. FIG. 6 is also an observation image of an example. The example will be described below.

Pillar dense area 11 illustrated in FIG. 6 acts as a filter. Pillar dense area 11 prevents impurities in the blood, such as insoluble components larger than blood cells, from entering a channel part downstream of pillar dense area 11. In addition, if there are blood cells that are aggregated, pillar dense area 11 breaks the aggregated blood cells into individual blood cells. Pillar dense area 11 is provided so as to cross the blood flow in channel part 25a. Blood flows from the left (upstream) side to the right (downstream) side in the drawing, as indicated by the white arrow. Pillar dense area 11 connects the upper surface to the lower surface of channel part 25a. Each pillar has a shape such that the pillar does not provide great resistance to blood flow. That is, each pillar preferably has a shape that reduces resistance to a fluid, and can be, for example, a shape of a circle, ellipse, or a streamlined drop in plan view. FIG. 6 illustrates drop-shaped pillars as an example, but the present invention is not limited thereto. Further, pillar dense area 11 does not significantly reduce the blood flow rate.

In FIG. 6, channel part 25a includes section 12. Section 12 is adjacent to and downstream of pillar dense area 11. Section 12 includes few pillars. Channel part 25a further includes pillar dense area 13. Pillar dense area 13 is a next pillar dense area from pillar dense area 11 in the downstream direction as viewed from pillar dense area 11. The configuration of pillar dense area 13 may be substantially the same as that of pillar dense area 11.

In FIG. 6, section 12 is surrounded by pillar dense area 11, pillar dense area 13, and the sidewalls of channel part 25a, that is, the sidewalls of the microchannel. Channel part 25a further includes section 14. Section 14 is adjacent to and downstream of pillar dense area 13. Channel part 25a further includes a pillar dense area downstream of section 14. Elements with reference numerals 15 to 17 will be described in Examples.

7. Classification Process of Floating Cells in Microchannel

The floating cells contained in the blood that has passed through the pillar dense areas provided in channel part 25a are then classified in channel parts 25b to 25d. In the following, classification process of the floating cells by a microchannel will be described with reference to FIG. 7.

FIG. 7 illustrates microchannel 20 in plan view. The drawing schematically illustrates the process of classification of floating cells by using microchannel 20. For simplifying the explanation, the drawing illustrates branch channel 26a with ten small channels, and branch channel 26b with three small channels.

As illustrated in FIG. 7, blood BL continuously flows from channel part 25a (not illustrated in FIG. 7) located further upstream of channel part 25b. Blood BL contains a large amount of cells. The flow of clarified liquid CL is continuously brought into contact with the flow of blood BL from a lateral side of the blood flow. The cells flowing through main channel 23 are thus continuously pushed from the side of main channel 23. As a result, the flow of blood BL is continuously pushed toward the opposite side of the flow of clarified liquid CL. In the channel parts 25b to 25c, floating cells are continuously pushed toward the side of branch channels 26a and 26b, and the floating cells continuously flow into these branch channels.

As illustrated in FIG. 7, non-nucleated red blood cells 27 continuously flow into branch channel 26a. In channel part 25c, non-nucleated red blood cells 27 in blood BL are hydraulically classified. The classification is continuously performed in the flow of blood BL on the side that is not in contact with the flow of clarified liquid CL.

As illustrated in FIG. 7, branch channel 26a functions as a channel for removing non-nucleated red blood cells 27. The diameter of the inscribed circle (hereinafter, also referred to as "inscribed diameter") of a small channel of branch channel 26a is 12 to 19 $\mu$m. The inscribed diameter may be any of 13, 14, 15, 16, 17, and 18 $\mu$m.

As illustrated in FIG. 7, nucleated cells 29a to 29c continuously flow into branch channel 26b. In channel part 25c downstream of channel part 25b, nucleated cells 29a to 29c in blood BL are hydraulically classified. The classification is continuously performed in the flow of blood BL on the side that is not in contact with the flow of clarified liquid CL. A cell suspension of nucleated cells is continuously obtained, in particular, from branch channel 26b.

As illustrated in FIG. 7, branch channel 26b functions as a channel for collecting nucleated cells 29a to 29c. The inscribed diameter of a small channel of branch channel 26b is 20 to 30 $\mu$m. The inscribed diameter may be any of 21, 22, 23, 24, 25, 26, 27, 28, and 29 $\mu$m. The diameter of nucleated cells such as nucleated red blood cells is considered to be 11 to 13 $\mu$m.

The inscribed diameter of each small channel of branch channels 26a and 26b illustrated in FIG. 7 is the diameter of an inscribed circle in a cross section orthogonal to the small channel. The shape of the cross section of the small channel may be rectangular, other polygonal, or circular. Substantially the same configuration applies to other branch channels. The floating cells and blood plasma that have not been taken into branch channel 26a or 26b continuously pass through channel part 25d as flow-through FT. Those floating cells and blood plasma are then reach outlet 22c in FIG. 1. For example, flow-through FT includes aggregated blood cells.

As described above, a fluid containing no floating cells of a certain size or larger can be collected from branch channel 26a. As a result, non-nucleated red blood cells 27 are classified in this procedure. Further, nucleated cells 29a and other nucleated cells are classified in the downstream channel.

The above description explains a series of procedures for classifying by using the blood separation device according to the embodiment.

Examples

In this example, an Example and Comparative Examples in which the effect of the column on eliminating or reducing clogging in a microchannel is investigated and the effects thereof will be described with reference to FIGS. 6, 8, and 9.

The series of steps 1 to 5 of this example is as follows.

1. Cutting off small particles contained in beads that are porous particles
2. Spiking blood with fluorescent particles to obtain sample blood
3. Allowing the blood to flow into a blood separation device to be classified
4. After the classification, collecting F1 to F3 fractions and evaluating the collection rate of the fluorescent particles
5. Evaluating the clogging rate of a blood cell separation chip during the classification

Descriptions of steps 1 to 3 are for the classification performed in the examples. Step 4 is for the evaluation of data obtained by the classification. Step 5 is for the evaluation of data obtained during the classification.

1.1 Cutoff

In this example, small particles were cut off from porous particles. In this example, beads Sepharose CL-6B were used as the porous particles. Sepharose is a trademark. The gel matrix of Sepharose CL-6B is composed of spheres made of 6% crosslinked agarose. The particle size before the cutoff was 45 to 165 $\mu$m. Into a nylon cell strainer (FALCON, trademark), 1 mL of the beads are placed. The beads were filtered while stirred with a stirrer overnight. The filtration time may be several hours to 24 hours. The filtration was performed in distilled water. The mesh size of the cell strainer was 40 $\mu$m, 70 $\mu$m, or 100 $\mu$m. The filtration was performed for the cell strainer of each mesh size. In this example, an example that uses the 70 $\mu$m mesh cell strainer will be described in detail. The mesh size of a cell strainer corresponds to the cutoff diameter. Large particles filtered by the cutoff were housed in a column to be used for pretreatment. The smaller the mesh size, the larger the number of particles filtered on the mesh. The beads were washed twice with PBS.

Beads were suspended in a clarified liquid in the same volume as that of the beads. Added was 20 $\mu$L of bead suspension to 2 mL of the clarified solution and obtained mixture was well mixed. The clarified solution is a PBS-based buffer. The mixed solution of the beads and the clarified liquid was further added to a clarified liquid. By pouring a part of the clarified solution into the blood cell separation chip in advance, the inside of the blood cell separation chip was immersed in the buffer solution in advance. The immersion of the blood cell separation chip was performed for 40 minutes.

1-2. Evaluation of Cutoff Diameter

The blood cell separation chip after the classification was observed with a microscope. When 100 $\mu$m beads were placed in the column and blood was pretreated, debris was observed inside channel part 25a and in the vicinity of inlet 21a. The channel was slightly clogged by the debris. On the other hand, no debris was observed when beads having a cutoff diameter of 70 $\mu$m or 40 $\mu$m was used for the pretreatment. Further, the classification of the sample blood was continued for 2 hours and 40 minutes, but no clogging of the channel was observed. It was found that the cutoff diameter of 70 $\mu$m or less is desirable in order to obtain the effect of preventing clogging of a channel caused by debris. The cutoff diameter may be 60 $\mu$m or 50 $\mu$m.

In all cases of cutoff diameters of 40 $\mu$m, 70 $\mu$m, and 100 $\mu$m, the entire amount of sample blood could be processed with a blood cell separation chip. There was no excess sample blood that could not be fractionated-the excess of the sample blood might be caused by clogging of the blood cell separation chip by debris. In the following tests, the cutoff diameter of 70 $\mu$m was employed.

The beads subjected to the cutoff were housed in the housing part of the column. For example, 50 $\mu$L of beads having a cutoff diameter of 70 $\mu$m were housed in the column.

2. Spiking of Blood with Fluorescent Particles

The "blood" in this example was whole blood collected from a healthy human adult. The blood collection tube to be used for collecting blood contains citric acid and EDTA, which prevent blood coagulation. Collecting a specified amount of blood into the blood collection tube automatically adds an appropriate amount of citric acid and EDTA to the blood. The whole blood on the third day stored at 4°C after blood collection was spiked with fluorescent particles to obtain sample blood. Herein, to "spike" means to add a small amount of cells or a substance imitating cells. "Fluorescent particles" are spherical particles that imitate fetal nucleated red blood cells, which are contained in maternal blood at a small amount. The fluorescent particles correspond to, for example, nucleated cells 29a to 29c in FIG. 5. A clarified liquid (a buffer solution based on PBS) was added to the obtained sample blood to dilute the mixture 5-fold. In this example, the blood was spiked with two types of fluorescent particles having different diameters. Specifically, the blood was spiked with resin fluorescent particles having a diameter of 8.42 $\mu$m and a diameter of 10 $\mu$m. FITC Particles (cat #F1CP-80-2) from Spherotech were used as fluorescent particles having a diameter of 8.42 $\mu$m. FluoSpheres (trademark) polystyrene microspheres, 10 $\mu$m, blue fluorescent (365/415), from Invitrogen were used as 10.0 $\mu$m fluorescent particles. The diluted blood in 1 mL was spiked with 60 fluorescent particles having a diameter of 8.42 $\mu$m and 60 fluorescent particles having a diameter of 10 $\mu$m, a total of 120 particles. Whole blood may be spiked with of fluorescent particles and then diluted. For example, 1 mL of whole blood may be spiked with 300 fluorescent particles having a diameter of 8.42 $\mu$m and 300 fluorescent particles having a diameter of 10 $\mu$m and then diluted 5-fold.

3. Classification by Blood Cell Separation Chip

The diluted sample blood was allowed to pass through the column for pretreatment, classified with a single layer blood cell separation chip, thereby collecting fluorescent particles. The results of the classification are described with reference to FIGS. 1 and 6. A large number of leukocytes containing lymphocytes were obtained in fraction F2. A large number of non-nucleated mature red blood cells were obtained in fraction F1. Most (> 99.9%) of the cells that have flowed into the blood cell separation chip flew out to fraction F1. Not many blood cells were obtained in fraction F3. The fluorescent particles are lymphocyte-like, and thus are expected to be contained in fraction F2.

The sample blood to be flowed in the Example, Comparative Example 1 and Comparative Example 2 is as described in "(2) Spike of Blood with Fluorescent Particles" above, and is from the same conditions. The sending rate of the sample blood from the syringe was 20 $\mu$L/min. The sending rate of the clarified liquid from the syringe was 40 $\mu$L/min. When 1.8 mL of the sample blood was sent, i.e., 90 minutes after the start of classification, fluorescent particles were collected from F1 to F3 fractions including the fluorescent particles, and the collection rate was determined.

The difference between the Example and Comparative Examples 1 and 2 is the configuration of the column of the blood separation device.

The column of the Example includes both CL-6B beads and a filter. The column is filled with 50 $\mu$L of CL-6B beads having a cutoff diameter of 70 $\mu$m. The mesh of the filter is grid-like and has a diameter of 20 $\mu$m.

Comparative Example 1 includes no column. That is, Comparative Example 1 does not include CL-6B beads or a filter. The sample blood was introduced directly into the blood cell separation chip.

The column of Comparative Example 2 is not filled with CL-6B beads. That is, the column of Comparative Example 2 includes only a filter and no CL-6B bead. The mesh of the filter is grid-like as in the Example and has a diameter of 20 $\mu$m.

4. Evaluation of Collection Rate of Fluorescent Particles

The collection rate of fluorescent particles can be obtained by using the following Expression 1.

$$\text{Collection rate of fluorescent particles (\%)} = \text{Number of fluorescent particles collected in a certain period of time} \,/\, \text{Expected value of the number of fluorescent particles contained in blood cells fractionated within the certain period of time} \times 100 \qquad \dots \text{Expression 1}$$

The "number of fluorescent particles collected in a certain period of time" is the number of fluorescent particles found by actual measurement.

The "expected value of the number of fluorescent particles contained in blood cells fractionated within the certain period of time" of Expression 1 can be obtained by using the following Expression 2.

$$\text{Expected value of the number of fluorescent particles contained in blood cells fractionated within the certain period of time (Number)} = (\text{Number of blood cells fractionated within the certain period of time (cells)} \,/\, \text{Blood cells per mL of whole blood (cells/mL)}) \times \text{Number of fluorescent particles per mL of whole blood at the time of adding fluorescent particles to 5-fold diluted blood (number/mL)} \qquad \dots \text{Expression 2}$$

The collection rates of the fluorescent particles of the Example and Comparative Examples 1 and 2 are shown in Table 1 below.

Table 1

| Collection rates of fluorescent particles of Example and Comparative Examples 1 and 2 | | | | |
|---|---|---|---|---|
| | CL-6B beads | Filter | Collection rate of fluorescent particles (%) | |
| | | | Diameter 8.42 μm | Diameter 10 μm |
| Example | + | + | 84.5 | 110.4 |
| Comparative Example 1 | - | - | 96.6 | 114.7 |
| Comparative Example 2 | - | + | 109.8 | 114.3 |

For the Example, the upper row of Table 1 shows the collection rate of fluorescent particles when sample blood was allowed to flow for 90 minutes with CL-6B beads having a cutoff diameter of 70 μm. The numbers of fluorescent particles found by the measurement in the fractions of F1 to F3 were 76 fluorescent particles having a diameter of 8.42 μm and 85 fluorescent particles having a diameter of 10.0 μm. The number of blood cells found in the fractions of F1 to F3 was $1.27 \times 10^9$. The number of blood cells per mL of the whole blood before adding fluorescent particles was $4.42 \times 10^9$. The numbers of fluorescent particles per mL of the whole blood at the time of adding the fluorescent particles to the 5-fold diluted blood were 313 fluorescent particles having a diameter of 8.42 μm and 268 fluorescent particles having a diameter of 10 μm. The collection rates of the fluorescent particles obtained from the above measured values and the above calculation expressions, namely Expression 1 and Expression 2, were 84.5% for fluorescent particles having a diameter of 8.42 μm and 110.4% for fluorescent particles having a diameter of 10 μm.

For Comparative Example 1, the middle row of Table 1 shows the collection rate of fluorescent particles when sample blood was allowed to flow for 90 minutes. The numbers of fluorescent particles found by the measurement in the fractions of F1 to F3 were 114 fluorescent particles having a diameter of 8.42 μm and 131 fluorescent particles having a diameter of 10.0 μm. The number of blood cells found in the fractions of F1 to F3 was $1.70 \times 10^9$. The number of blood cells per mL of the whole blood before adding fluorescent particles was $4.42 \times 10^9$. The numbers of fluorescent particles per mL of the whole blood at the time of adding the fluorescent particles to the 5-fold diluted blood were 307 fluorescent particles having a diameter of 8.42 μm and 297 fluorescent particles having a diameter of 10 μm. The collection rates of the fluorescent particles obtained from the above measured values and the above calculation expressions, namely Expression 1 and Expression 2, were 96.6% for fluorescent particles having a diameter of 8.42 μm and 114.7% for fluorescent particles having a diameter of 10 μm.

For Comparative Example 2, the lower row of Table 1 shows the collection rate of fluorescent particles when sample blood was allowed to flow for 90 minutes. The numbers of fluorescent particles found by the measurement in the fractions of F1 to F3 were 133 fluorescent particles having a diameter of 8.42 μm and 127 fluorescent particles having a diameter of 10.0 μm. The number of blood cells found in the fractions of F1 to F3 was $1.71 \times 10^9$. The number of blood cells per mL of the whole blood before adding fluorescent particles was $4.42 \times 10^9$. The numbers of fluorescent particles per mL of the whole blood at the time of adding the fluorescent particles to the 5-fold diluted blood were 313 fluorescent particles having a diameter of 8.42 μm and 289 fluorescent particles having a diameter of 10 μm. The collection rates of the fluorescent particles obtained from the above measured values and the above calculation expressions, namely Expression 1 and Expression 2, were 109.8% for fluorescent particles having a diameter of 8.42 μm and 114.3% for fluorescent particles having a diameter of 10 μm.

When CL-6B beads having a cutoff diameter of 70 μm were used, the collection rate of fluorescent particles, which were used as substitutes for fetal nucleated red blood cells, was 80% or more in every case, thus the collection rate is considered to be sufficiently high.

This example is performed as a model experiment for enrichment of fetal nucleated red blood cells. The results above indicate that the pretreatment with a column is useful for enriching fetal nucleated red blood cells. Specifically, it is indicated that long-term classification can be performed by preventing the clogging of the blood cell separation chip. A large amount of the sample blood can be processed by performing long-term classification by a method using the blood separation device of this example. This indicates that the amount of fetal nucleated red blood cells obtained per process is large.

5. Evaluation of Clogging Rate of Blood Cell Separation Chip

In the following, the evaluation of the clogging rate of the blood cell separation chip will be described with reference to FIGS. 6, 8, and 9.

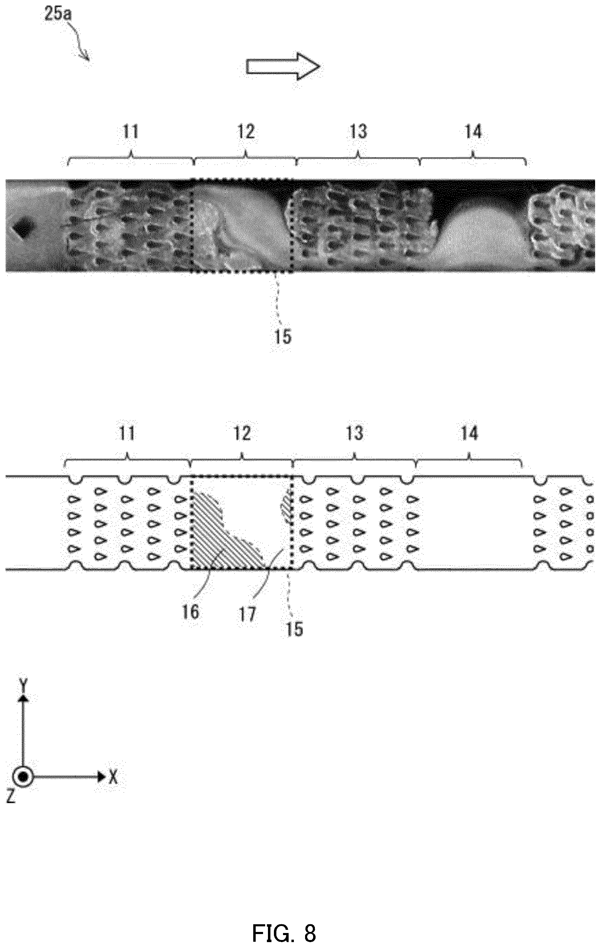

Before the classification is completed in "4. Evaluation of Collection Rate of Fluorescent Particles" above, acquired was the image data of microchannel 20 (blood cell separation chip, hereinafter referred to as "chip") of FIG. 1 in a state where blood is flowing through the chip. The clogging rate was evaluated by performing image analysis on the acquired image data. Specifically, main channel 23 (channel part 25a) of the chip of FIG. 1 was evaluated. The evaluation of channel part 25a in this example and Comparative Examples 1 and 2 was performed 30 to 90 minutes after the sample blood was started to flow. No clogging was observed in the Example, and clogging was observed in Comparative Examples 1 and 2. In the following, a specific method for evaluating the clogging rate will be described with reference to FIG. 8 showing Comparative Example 1 with clogging occurred.

FIG. 8 illustrates an observation image (upper row) of channel part 25a in Comparative Example 1 and the sketch thereof (lower row). FIG. 8 is an enlarged view of the portion shown by area VI in FIG. 1. The clogging rate was evaluated for section 12. A chip in which the sample blood was continuously flowing was placed under a USB camera (HOZAN TOOL IND. CO., LTD.). An observation image was acquired with the chip in plan view. The chip was photographed with HOZAN USB cam software to obtain the image data shown in the upper row of FIG. 8. The image data was read into ImageJ software and analyzed on the software.

Area 15 corresponding to section 12 was cut out from the image data in FIG. 8. The total number of pixels in area 15 is treated as the total area of section 12. Debris part 16 in area 15 was visually distinguished from flow part 17. Debris part 16 was occupied by debris accumulated starting from pillar dense area 11. "Debris" is a gel-like substance formed from precipitation-causing substance CC (see FIG. 5) contained in the sample blood. Such debris entering the inside of pillar dense area 11 causes clogging of pillar dense area 11. Debris part 16 pushes the flow of blood cells away. On the other hand, blood cells were flowing smoothly in flow part 17.

In FIG. 8, a line was drawn between debris part 16 and flow part 17 by visual observation. The total number of pixels of the portion of area 15 excluding debris part 16, that is, flow part 17, was obtained. This number of pixels was specified as the area of flow part 17. The area of flow part 17 was subtracted from the total area of area 15 to obtain the estimated area of debris part 16. This estimated area was used as the debris area. The debris area occupying the total area of section 12 was calculated as a percentage. This value served as a clogging rate.

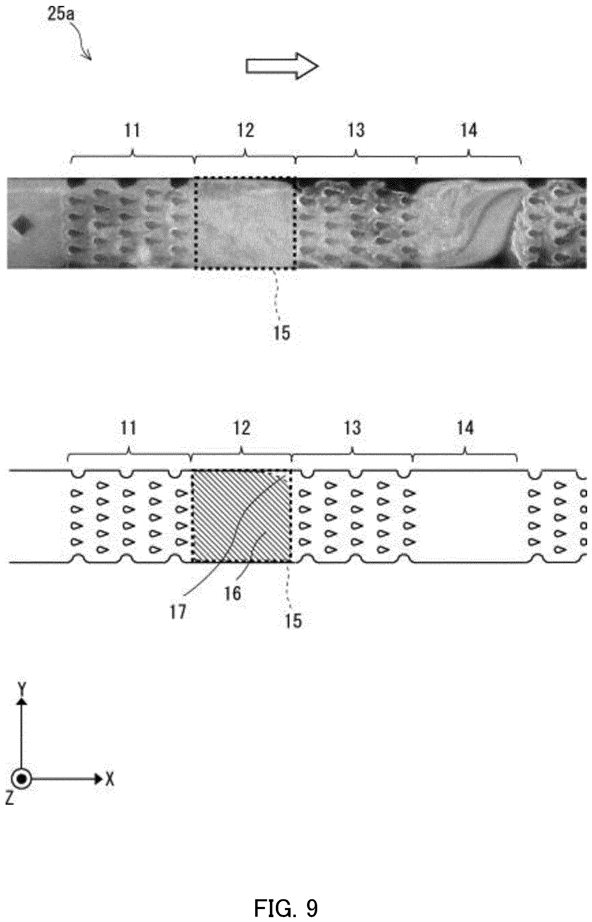

FIG. 9 illustrates an observation image (upper row) of channel part 25a in Comparative Example 2 and the sketch thereof (lower row). FIG. 6 illustrates an observation image (upper row) of channel part 25a in Example 1 and the sketch thereof (lower row).

As described above (see "4. Evaluation of Collection Rate of Fluorescent Particles"), the column conditions of Comparative Example 1, Comparative Example 2 and the Example are as follows.

Comparative Example 1 includes no column. That is, Comparative Example 1 does not include CL-6B beads or a filter. The sample blood was introduced directly into the blood cell separation chip.

The column of Comparative Example 2 is not filled with CL-6B beads. That is, the column of Comparative Example 2 includes only a filter and no CL-6B bead. The mesh of the filter is grid-like and has a diameter of 20 $\mu$m.

The column of the Example includes both CL-6B beads and a filter. The column is filled with 50 $\mu$L of CL-6B beads having a cutoff diameter of 70 $\mu$m. The mesh of the filter is grid-like and has a diameter of 20 $\mu$m.

The clogging rates of the blood cell separation chips of the Example and Comparative Examples 1 and 2 are shown in Table 2 below. The clogging rate was obtained at the time when the sample blood was flowing for 90 minutes.

Table 2

| Clogging rates of blood cell separation chip of Example and Comparative Examples 1 and 2 | | | |
|---|---|---|---|
| | CL-6B beads | Filter | Clogging rate of blood cell separation chip (%) |
| Example | + | + | 0.0 |
| Comparative Example 1 | - | - | 46.6 |
| Comparative Example 2 | - | + | 96.5 |

As shown in Table 2, the clogging rate was 46.6% in Comparative Example 1 not including CL-6B beads or a filter. In Comparative Example 2 with a column including only a filter and no CL-6B beads, the clogging rate increased to 96.5% and most of section 12 was clogged. This result indicates that the filter alone does not prevent clogging. FIGS. 8 and 9 show that the debris was generated in such a way that the debris is connected to pillar dense area 11, thereby causing clogging in each comparative example. Further, the clogging rate of Comparative Example 2 being higher than that of Comparative Example 1 leads to the assumption that the filter is likely to generate a precipitation-causing substance causing debris.

In contrast, no debris was observed in the Example with a column including both CL-6B beads and a filter, and the clogging rate was 0.0%. The above results shows that the column provided with CL-6B beads and at least one filter can prevent the generation of debris. A blood separation device suitable for eliminating or reducing clogging thus can be provided. In addition, the blood used in this example and the comparative examples was blood 3 days after blood collection. Even with such blood, it is possible to collect rare cells such as fetal nucleated red blood cells by using a small amount of beads.

Reference Example

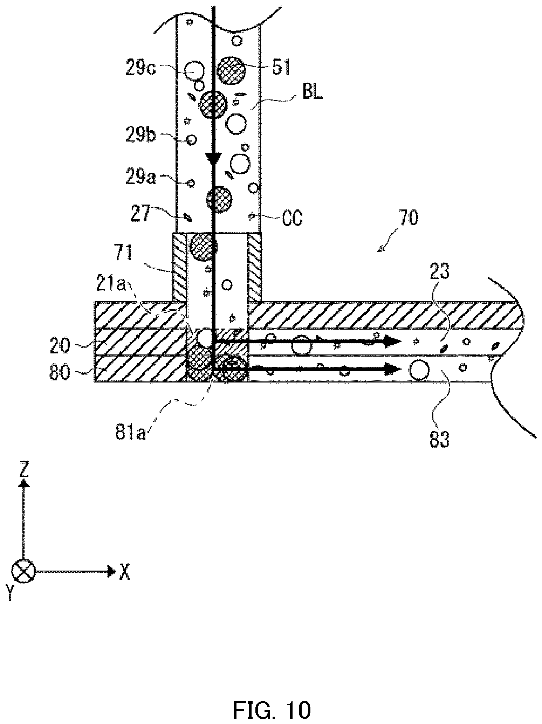

FIG. 10 illustrates a reference example in which classification is performed without using a column. FIG. 10 is a partial front cross-sectional view illustrating a state in which porous material 51 is poured into microchannel 20 together with blood BL. Blood BL contains non-nucleated red blood cells 27, nucleated cells 29a to 29c and precipitation-causing substance CC. Porous material 51 interacts with, for example, precipitation-causing substance CC, thereby providing an effect of eliminating or reducing clogging in microchannel 20.

In FIG. 10, microchannel 80 is the lower microchannel illustrated in FIG. 5. Microchannel 80 includes inlet 81a and main channel 83. Inlet 81a is substantially the same as inlet 21a of microchannel 20. Main channel 83 is substantially the same as main channel 23 of microchannel 20.

In FIG. 10, the blood flow is indicated by black arrows. Blood BL enters main channel 23 via inlet 21a of microchannel 20. At the beginning of the flow of blood BL, porous material 51 gathers at inlet 81a, and not at inlet 21a. Porous material 51 does not enter main channel 23 or main channel 83. That is, porous material 51 filling inlet 81a interacts with blood BL at the beginning of the flow of blood BL. On the other hand, as porous material 51 does not fill inlet 21a, the effect of eliminating or reducing clogging by porous material 51 may not be provided at inlet 21a. In other words, when the amount of porous material 51 poured together with blood BL is small, the effect of the porous material 51 on eliminating or reducing clogging in microchannel 20 may vary in the upper part of channel chip 70. In contrast, in the Example with a column including both CL-6B beads and filters, blood BL reacts with the CL-6B beads before blood BL enters main channel 23, thus the precipitation-causing substance can be removed before the classification. Therefore, the blood separation device provided with the column according to this example is advantageous especially when the amount of beads is small.

The present invention is not limited to the above embodiment, and can be appropriately modified without departing from the scope thereof. For example, the blood cell separation device may be another device for blood. In one example, the device for blood includes a column and a microchannel located downstream of the column. The column includes a porous material as a stationary phase. Blood brought into contact with the porous material flows into the microchannel. In one example, the device for blood is a blood analysis device. Blood is physically or chemically analyzed in a microchannel.

This application claims priority to Japanese Patent Application No. 2019-192420, filed on October 23, 2019, the disclosure of which is incorporated herein by reference in its entirety.

Reference Signs List

1 Blood cell separation device
11 Pillar dense area
12 Section
13 Pillar dense area
14 Section
15 Area
16 Debris part
17 Flow part
20 Microchannel
21a, 21b Inlet
22a to 22c Outlet
23 Main channel
25a to 25d Channel part
26a, 26b Branch channel

27 Non-nucleated red blood cell
28 Junction
29a to 29c Nucleated cell
30 Syringe
35 Syringe
50 Column
51 Porous material
52 Housing part
53a, 53b Filter
54 Coupling part
55 Coupling part
56 Tube
60 Column body
70 Channel chip
71 Inlet
72 Outlet
80 Microchannel
81a Inlet
83 Main channel
BL Blood
CC Precipitation-causing substance
CF Cutoff diameter
CL Clarified liquid
F1 to F3 Fraction

**Claims**

1. A device for blood, the device comprising:

   a column; and
   a microchannel located downstream of the column, wherein:

      the column includes a porous material as a stationary phase, and
      blood flows through the microchannel after contacted with the porous material.

2. The device for blood according to claim 1, wherein:

   the porous material comprises particles;
   the column further includes a housing part and a filter, wherein the housing part is for housing the porous material, the filter is for trapping the particles of the porous material, and the filter is located downstream of the housing part on one side of the housing part, the one side being closer to the microchannel than the other side of the housing part is;
   a small particle having a particle size equal to or smaller than a cutoff diameter is removed in advance from the particles of the porous material; and
   an opening of the filter is smaller than the cutoff diameter.

3. The device for blood according to claim 2, wherein the cutoff diameter is in a range of 25 $\mu$m to 100 $\mu$m.

4. The device for blood according to claim 2 or 3, wherein a diameter of a mesh of the filter is in a range of 20 $\mu$m to 40 $\mu$m, and the range is less than the cutoff diameter.

5. The device for blood according to any one of claims 2 to 4, wherein:

   the particles of the porous material have a particle size distribution; and
   median particle size (d50V) of the particles of the porous material in a volume-based cumulative distribution is 25 to 280 $\mu$m, wherein the particle size distribution represents a particle size distribution before the small particle is removed according to the cutoff diameter.

6. The device for blood according to any one of claims 2 to 5, wherein:
the column further includes a filter for trapping the particles of the porous material, the filter being located upstream of the housing part on the other side of the housing part, the other side being farther from the microchannel than the one side of the housing part is.

7. The device for blood according to any one of claims 1 to 6, wherein:
when the blood flows through the microchannel after contacted with the porous material, the microchannel hydraulically classifies blood cells in the blood.

8. The device for blood according to claim 7, wherein:

the device for blood is for blood cell separation;
the microchannel is made of a flat chip, the flat chip including a pillar dense area and a hydraulic channel located downstream of the pillar dense area;
the column is connected to a front or a back of the flat chip; and
the device for blood further includes an outlet for discharging the hydraulically classified blood cells from the microchannel to an outside of the device for blood.

9. The device for blood according to any one of claims 1 to 8, wherein:

the porous material comprises particles; and
the particles of the porous material have a porous surface made of a polysaccharide, silica, or a resin.

10. A method for using the device for blood according to any one of claims 1 to 9, the method comprising: allowing blood to enter the microchannel from the column, wherein

the column and the microchannel are provided as separate bodies in the device for blood,
the column includes a coupling part,
the microchannel includes an inlet, and
the blood is allowed to enter the microchannel after the coupling part is coupled with the inlet to integrate the column with the microchannel.

FIG. 1

FIG. 2

FIG. 3

FIG. 4

FIG. 5

FIG. 6

FIG. 7

25a

11    12    13    14

15

11    12    13    14

16    17    15

Y

Z    X

FIG. 8

FIG. 9

FIG. 10

<table>
<tr><td colspan="2"><strong>INTERNATIONAL SEARCH REPORT</strong></td><td colspan="2">International application No.<br>PCT/JP2020/038986</td></tr>
</table>

**A. CLASSIFICATION OF SUBJECT MATTER**
C12M 3/06(2006.01)i; B01D 15/00(2006.01)i; B01J I9/00(2006.01)i; G01N 33/48(2006.01)i
FI: G01N33/48 B; B01J19/00 321; B01D15/00 M; C12M3/06
According to International Patent Classification (IPC) or to both national classification and IPC

**B. FIELDS SEARCHED**

Minimum documentation searched (classification system followed by classification symbols)
C12M3/06; B01D15/00; B01J19/00; G01N33/48

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/078277 A1 (TL GENOMICS INC.) 25 April 2019 (2019-04-25) claims, paragraphs [0030]-[0058], fig. 1-2, 12, 16 | 1-10 |
| Y | JP 2016-502653 A (THE REGENTS OF THE UNIVERSITY OF CALIFORNIA) 28 January 2016 (2016-01-28) paragraphs [0043], [0078], fig. 9 | 1-10 |
| Y | JP 1-229972 A (FUSO PHARMACEUTICAL INDUSTRIES, LTD.) 13 September 1989 (1989-09-13) page 4, lower left column, line 11 to lower right column, line 10 | 1-10 |
| Y | JP 20I6-527494 A (UNIVERSITY OF WASHINGTON THROUGH ITS CENTER FOR COMMERCIALIZATION) 08 September 2016 (2016-09-08) paragraphs [0093]-[0094] | 8-10 |
| Y | JP 2007-71711 A (FUJIFILM CORPORATION) 22 March 2007 (2007-03-22) paragraphs [0040]-[0044], [0048] | 8-10 |

☒ Further documents are listed in the continuation of Box C.    ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 December 2020 (03.12.2020) | 15 December 2020 (15.12.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2020/038986

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2013-17429 A (KONICA MINOLTA HOLDINGS, INC.) 31 January 2013 (2013-01-31) entire text all drawings | 1-10 |
| A | US 2015/0079601 A1 (MERIDIAN BIOSCIENCE, INC.) 19 March 2015 (2015-03-19) entire text all drawings | 1-10 |
| A | HUANG, R. et al., "A microfluidics approach for the isolation of nucleated red blood cells (NRBCs) from the peripheral blood of pregnant women", PRENATAL DIAGNOSIS, 2008, 28, 892-899 entire text all drawings | 1-10 |
| A | MIZUNO, M. et al., "Magnetophoresis-Integrated Hydrodynamic Filtration System for Size-and Surface Marker-Based Two-Dimensional Cell Sorting", analytical chemistry, June 2013, 85, 7666-7673 entire text all drawings | 1-10 |
| A | SAJEESH, P. et al., "Characterization and sorting of cells based on stiffness contrast in a microfluidic channel", RSC Advances, 2016, 6, 74704-74714 entire text all drawings | 1-10 |

Form PCT/ISA/210 (continuation of second sheet) (January 2015)

International application No.

PCT/JP2020/038986

| Patent Documents referred in the Report | Publication Date | Patent Family | Publication Date |
|---|---|---|---|
| WO 2019/078277 A1 | 25 Apr. 2019 | CA 3076601 A | |
| JP 2016-502653 A | 28 Jan. 2016 | WO 2014/113110 A2 paragraphs [0068], [0106], fig. 9 US 2014/0113324 A1 US 2014/0315287 A1 US 2015/0355073 A1 EP 2912434 A CA 2920132 A | |
| JP 1-229972 A | 13 Sep. 1989 | (Family: none) | |
| JP 2016-527494 A | 08 Sep. 2016 | US 2016/0146823 A1 paragraphs [0119]-[0120] WO 2015/002975 A1 EP 3017308 A TW 201516412 A CN 105518464 A CN 110988373 A | |
| JP 2007-71711 A | 22 Mar. 2007 | (Family: none) | |
| JP 2013-17429 A | 31 Jan. 2013 | (Family: none) | |
| US 2015/0079601 A1 | 19 Mar. 2015 | WO 2013/177525 A2 EP 2854981 A AU 2013266076 A CA 2873323 A1 DK 2854981 T ES 2689441 T SI 2854981 T PL 2854981 T PT 2854981 T | |

Form PCT/ISA/210 (patent family annex) (January 2015)

**EP 4 050 093 A1**

**Patent documents cited in the description**

- WO 2018123220 A

- JP 2019192420 A